Europäisches Patentamt
European Patent Office
Office européen des brevets

(19)

(11) Publication number: **0 490 407 A2**

(12) **EUROPEAN PATENT APPLICATION**

(21) Application number: **91121440.1**

(51) Int. Cl.$^5$: **C12P 41/00**, C12P 11/00

(22) Date of filing: **13.12.91**

(30) Priority: **14.12.90 IT 2239890**

(43) Date of publication of application:
**17.06.92 Bulletin 92/25**

(84) Designated Contracting States:
**AT BE CH DE ES FR GB LI LU NL SE**

(71) Applicant: **MINISTERO DELL' UNIVERSITA' E DELLA RICERCA SCIENTIFICA E TECNOLOGICA**
**76, Lungotevere Thaon di Revel**
**I-00196 Roma(IT)**

(72) Inventor: **Bianchi, Daniele**
**59, Via Mose' Bianchi**
**I-20149 Milan(IT)**
Inventor: **Bosetti, Aldo, Dr.**
**6, Corso Italia**
**I-13100 Vercelli(IT)**
Inventor: **Cesti, Pietro, Dr.**
**51, Via Torino**
**I-28069 Trecate, Novara(IT)**
Inventor: **Spezia, Sandro, Dr.**
**7, Via San Francesco**
**I-29100 Piacenza(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

(54) **Process for the enzymatic separation of the optical isomers of tosyloxy-alkanols.**

(57) Described is a process for the biotechnological resolution, by means of stereoselective enzymatic esterification, of the racemic mixture of optical isomers of tosyloxy-alkanols of general formula (I):

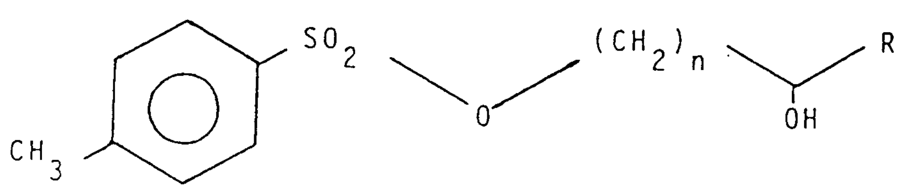

(I)

wherein R represents an alkyl, haloalkyl or $C_2$-$C_{10}$ alkenyl group, either linear or branched, and n is 1 or 2, by using aliphatic acid anhydrides in the presence of an enzyme which is capable of selectively catalyzing the esterification of the (R) isomer, leaving the (S) isomer basically unchanged.

EP 0 490 407 A2

The present invention relates to a process for the stereoselective or asymmetric enzymatic separation of the optical isomers of racemic 1-tosyloxy-alkanols.

More specifically, the present invention relates to a biotechnological process for the enzymatic separation or resultion of racemic mixtures of the optical isomers of alcohols of general formula (I):

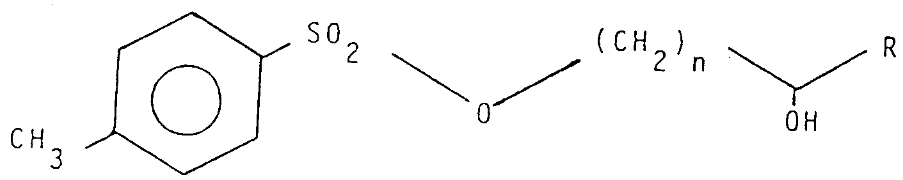

(I)

wherein R represents a linear or branched alkyl, haloalkyl or $C_2$-$C_{10}$ alkenyl group and n is 1 or 2, by means of stereoselective asymmetric esterification with acylating agents selected from aliphatic acid anhydrides in the presence of specific enzymes.

The above alcohols, in the form of the pure enantiomers, can be used as intermediates in the synthesis of optically active epoxides, e.g. according to the following reactive scheme:

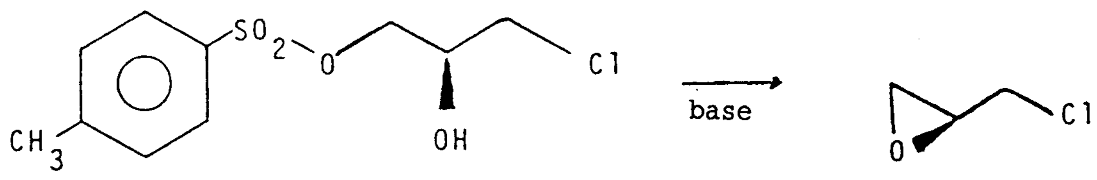


The above epoxides, in turn, are important chiral reagents for the preparation of pharmaceuticals (beta-blockers, antibiotics), agrochemicals (pheromones) and chiral polymers.

It is therefore highly desirable to have available an effective method for separating the optically active forms of racemic compounds of general formula (I).

Direct synthetic procedures for the selective preparation of optically active 1-tosyloxy-2(3)-alkanols are already known. For example, these derivatives can be synthesized from the respective enantiomerically pure alpha-hydroxy acids (obtained by fermentation) by means of esterification, reduction with hydride and subsequent tosylation (Helvetica Chim. Acta 60, 1175, 1977).

For example, optically active 1-tosyloxybutan-3-ol (n = 2) can be prepared by starting from optically active 3-hydroxybutyric acid which is obtainable by stereoselective microbiological reduction of 3-keto-butyrate (J. Org. Chem. 1982, 47, 3850) or by hydrolysis of polyhydroxybutyrate of natural origin (Liebigs Ann. Chem. 1990, 513). The hydroxy group of said acid is then protected, reduced with hydride, deprotected and then transformed into the compound of formula (I) by means of tosylation of the primary hydroxy group. These syntheses, however, require the use of large quantities of expensive chemicals, they have no general applicability as not all of the alphahydroxy acids are available in both optical forms and, moreover, they require a large number of steps, with consequently reduced industrial yields.

A process for the preparation of optically active 1-tosyloxy-2-alkanols via the stereoselective enzymatic hydrolysis of a corresponding racemic ester has also been proposed (EP-A-197 484). This process involves the initial acylation of the 1-tosyloxy-2-alkanol, with a corresponding reduction of the yields.

Therefore there is a need for a simple, effective and economical method for the resolution of racemic compounds of formula (I) into the corresponding optical isomers.

Accordingly, object of the present invention is a process for the separation (resolution) of optical isomers of racemic mixtures of compounds of formula (I), which is simple, effective and economical and affords a high degree of optical purity.

It has now been found that this objective can be achieved by means of a biotechnological process of stereoselective or asymmetric enzymatic esterification of compounds of formula (I), starting from their racemic mixtures and using specific acylating agents in the presence of enzymes having a selective activity, which will be defined in more detail below.

In practice, enzymes belonging to the group of lipases are used, which are capable of stereoselectively catalyzing the esterification reaction of the (R)-alcohols of formula (I), leaving the (S) form practically unchanged.

The present invention, consequently, relates to a process for the enzymatic separation (resolution) of racemic mixtures of the optical isomers of tosyloxy-alkanols of general formula (I):

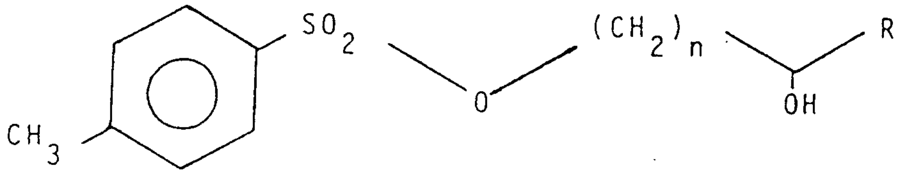

(I)

where R represents an alkyl, haloalkyl or $C_2$-$C_{10}$ alkenyl group and n is 1 or 2, wherein said racemic mixtures are reacted, in an organic solvent, with an anhydride of a saturated aliphatic acid of general formula (II):

$(R^1\text{-}CO)_2O$ (II)

where $R^1$ represents a linear or branched $C_1$-$C_6$ alkyl group, usually at temperatures ranging from 0 to 50 ° C and preferably at from 20 to 30 ° C, in the presence of a free or immobilized lipase deriving form Pseudomonas or Cromobacterium and capable of selectively causing the esterification reaction of the (R) isomer, leaving the (S) isomer of the starting racemic mixture basically unaffected, whereafter the above compounds may be separated by known procedures.

Examples of groups R are $C_1$-$C_{20}$, particularly $C_1$-$C_{10}$ alkyl, such as methyl, ethyl, propyl, butyl, pentyl, hexyl and octyl, which optionally carry one or more halogen atoms. Other examples of R are vinyl, propenyl and butenyl.

When the group R is haloalkyl, the halogen is preferably selected from chlorine and bromine.

The ester in (R) form and the alcohol in (S) form can be separated, as indicated above, by using conventional methods.

More specifically, as a brief summary of the process of the present invention, the racemic mixture of alcohols of formula (I) is reacted, in the presence of lipase derived form Pseudomonas of Cromobacterium, in an organic solvent, with an anhydride of formula (II) in accordance with the following reaction scheme:

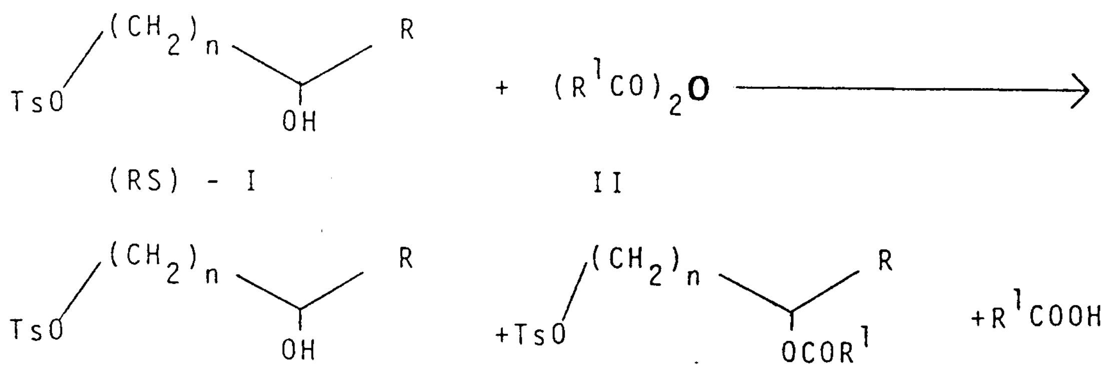
(RS) - I
II
(S)-I
(R)-III

where

Ts =

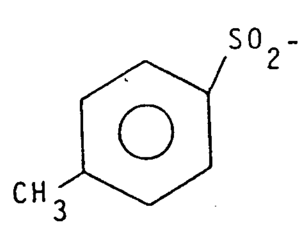

and the symbols R, $R^1$ and n have the above-defined meanings.

Surprisingly the use of acylating agents different from the anhydrides of formula (II) does not yield

results which are acceptable from an industrial point of view (deactivation of the enzyme and longer reaction times).

The preferred anhydrides of formula (II) are acetic anhydride and propionic anhydride. The reaction is carried out in an organic solvent, preferably selected from aromatic hydrocarbons and halogenated aliphatic hydrocarbons, for example, benzene, toluene, methylene chloride etc.

The racemic alcohols of formula (I) and the anhydrides of formula (II) are known compounds and/or can be synthesized by using conventional methods.

In the above reaction, the molar ratio of anhydride (II) to alcoholic substrate (I) usually ranges from 0.6:1 to 5:1, preferably from 0.8:1 to 1.5:1, and the enzyme is generally used in a weight ratio enzyme/substrate of formula (I) of from 1:1 to 1:100, preferably from 1:2 to 1:20.

The concentration of racemic alcohol of formula (I) in the reaction mixture usually ranges from 0.01 M to 2 M, depending on the racemic compound of formula (I) used.

The acylation or esterification may be carried out by vigorously stirring the reaction mixture of alcohol (I), anhydride (II), solvent and enzyme, either free or supported, as specified hereinafter, at temperatures of from 0 to 50 ° C, preferably of form 20 to 30 ° C.

Depending on the reaction conditions used, the reaction may be completed within 30 minutes to 60 hours; normally from 6 to 8 hours are sufficient.

At the end of the reaction the solid phase, basically composed of the enzyme, is filtered off. This can then be recovered and recycled without any substantial loss of activity.

After elimination of the excess anhydride (II), e.g. with an aqueous solution of alkali metal carbonate, the alcohol (I) in (S) form and the ester (III) in (R) form may be separated from the filtrate by using conventional methods such as column chromatography and fractionated distillation.

The enzymes used in accordance with the present invention belong to the group of lipases of microbic origin. The following enzymes are particularly active:

| Enzyme | Origin | Available from |
|---|---|---|
| LPL | Pseudomonas aeruginosa | Amano Pharm.Co.(Japan) |
| Lipasis P | Pseudomonas fluorescens | " |
| Lipasis CES | Pseudomonas sp. | " |
| Lipasis | Cromobacterium | Tojobo (Japan) |

In accordance with the present invention, the enzymes used may be free or immobilized on suitable supports, to increase their activity and stability and to facilitate their recovery and reuse.

Particularly suitable for this purpose are porous supports with a large surface area, such as Celite®, porous glass, silica, etc.

The immobilization can easily be carried out by absorbing a buffered aqueous solution of the enzyme on the support and then drying it.

The present process, thanks to its simple operating conditions, is highly advantageous. One of its particularly interesting aspects is that it can be carried out as a one-step process which leads to the direct separation of the enantiomers of the compounds of formula (I) with high yields and a high degree of purity (98% or more).

The abbreviation "e.e." stands for "enantiomeric excess".

The following examples provide a further illustration of the process of the present invention without limiting it in any way.

EXAMPLE 1

Immobilization of the enzyme

1.5 g of enzyme (Lipasis P from Pseudomonas fluorescens, Amano Pharm. Co., Ltd., 30 units per mg) dissolved in 4 ml of Na/K-phosphate (0.1 M at pH = 7) buffer solution are added to 5 g of Celite® 577 (Johns-Manville Ltd., Richmond, Surrey).

The mixture thus obtained is mixed so as to afford a uniform distribution of the enzyme and is then air-dried at 25 ° C for 24 hours.

Separation of the enantiomers of 1-tosyloxy-pronan-2-ol

300 mg of Celite® 577 containing 70 mg of immobilized Lipasis P enzyme and 570 mg of propionic anhydride are added to 1 g of (R,S) 1-tosyloxy-propan-2-ol dissolved in 25 ml of toluene.

The mixture is vigorously stirred at 25 °C and the reaction is followed by means of HPLC chromatography.

After 2 hours (55% conversion) the enzyme is recovered by filtration, while the organic phase is washed wiht a 5% aqueous solution of sodium carbonate. The toluene, after having been dried on sodium sulphate, is evaporated at reduced pressure and the residue is subjected to chromatography on a silica gel column, using as eluent a mixture of hexane-ethyl acetate (first 95:5 V/V, and then with 70:30 V/V).

In this way there are obtained 400 mg of (S)-1-toxyloxy-propan-2-ol as crystalline solid, m.p. = 52 °C, with $[\alpha]_D^{25}$ = +11.6 (c=1, $CHCl_3$), ee≧ 98%, $^1$H-NMR (200 MHz, $CDCl_3$), $\delta$ (ppm): 1.1 (3H, d), 2.4 (3H, s), 2.7 (1H, s), 3.65 and 3.9 (3H, m), 7.35 and 7.8 (4H, 2d); and 510 mg of (R)-2-propanoyloxy-1-tosyloxy propane as colourless oil with $[\alpha]_D^{25}$ = +10.4 (c=1, $CHCl_3$), ee = 79%, $^1$H-NMR (200 MHz, $CDCl_3$) $\delta$ - (ppm): 1.05 (3H, t), 1.1 (3H, d), 2.2 (2H, q), 2.38 (3H, s), 3.95 (2H, dd), 5 (1H, m), 7.35 and 7.75 (4H, 2d).

The enantiomeric excess and the configuration of both the alcohol and the ester (after conversion into the alcohol by means of hydrolysis in methanol) were determined by HPLC (Chiralcel® OC-Daicel® column).

EXAMPLE 2

Separation of the enantiomers of 1-tosyloxy-butan-2-ol 0.75 g of propionic anhydride and 300 mg of Celite® 577, containing 70 mg of Lipasis P enzyme immobilized as in example 1, are added to 2 g of (R,S)-1-tosyloxy-butan-2-ol dissolved in 50 ml of toluene.

The mixture is vigorously stirred for 2 hours at 25 °C, and the reaction is followed with HPLC.

At the end of the reaction (conversion 60%) the enzyme is separated by filtration, while the organic phase is washed with a 5% aqueous solution of sodium carbonate. After drying on sodium sulphate and evaporating the solvent at reduced pressure, the residue is subjected to chromatography on a silica gel column using hexane-ethyl acetate (9:1 V/V) as eluent.

In this way there are obtained 750 mg of (S)-1-tosyloxy butan-2-ol as crystalline solid, m.p. = 57-58 °C, with $[\alpha]_D^{25}$ = +10.5 (c=1, $CHCl_3$), ee> 98%, $^1$H-NMR (200 MHz, $CDCl_3$), $\delta$ (ppm): 0.95 (3H, t), 1.4 (2H, m), 2.0 (1H, s), 2.38 (3H, s), 3.6 - 4.1 (3H, m), 7.35 and 7.8 (4H, 2d); and 1.05 g of (R)-2-propanoyloxy-1-tosyloxy propane as colourless oil, $[\alpha]_D^{25}$ = +12.36 (c=1, $CHCl_3$), ee = 52%, $^1$H-NMR (200 MHz, $CDCl_3$) $\delta$ (ppm): 0.95 (3H, t), 1.1 (3H, t), 1.6 (2H, m), 2.2 (2H, q), 2.38 (3H, s), 4.1 (2H, m), 4.9 (1H, m), 7.35 and 7.75 (4H, 2d). The enantiomeric excess of these compounds was determined as in example 1.

EXAMPLE 3

Separation of the enantiomers of 1-tosyloxy-butan-2-ol

Using the same procedure and with the same quantities as in example 2, the enantiomers of (R,S)-1-tosyloxy butan-2-ol were separated, stopping the reaction after 30 minutes (30% conversion).

In this way, there were obtained 1.2 g of (S)-1-tosyloxy butan-2-ol with $[\alpha]_D^{25}$ = +33.2 (c=1, $CHCl_3$), ee = 30%; and 350 mg of (R)-propanoyloxy-1-tosyloxy-butane with $[\alpha]_D^{25}$ = +23.3 (c=1, $CHCl_3$), ee = 97%. For determining the enantiomeric excess and for other experimental details, reference is made to example 2.

EXAMPLE 4

Separation of the enantiomers of 1-tosyloxy-hexan-2-ol

0.5 g of propionic anhydride an 0.7 g of Celite® 577, containing 180 mg of Lipasis P enzyme immobilized as in example 1, are added to 1 g of (R,S)-1-tosyloxy-hexan-2-ol dissolved in 25 ml of toluene.

After 16 hours (52% conversion) the enzyme is recovered by filtration and the organic phase is washed with a 5% aqueous solution of sodium carbonate.

After drying and evaporating the solvent at reduced pressure, the residue is separated by column chromatography using hexane-ethyl acetate (9:1 V/V) as eluent. In this way, there are obtained 430 mg of

(S)-1-tosyloxy-hexan-2-ol as colourless oil with $[\alpha]_D^{25}$ = +7.3 (c=1, $CHCl_3$), ee = 91%, $^1$H-NMR (200 MHz, $CDCl_3$), $\delta$ (ppm): 0.9 (3H, t), 1.1-1.4 (6H, m), 2.1 (1H, s), 2.4 (3H, s), 3.7 (2H, m), 4.05 (1H, m), 7.4 and 7.7 (4H, 2d); and 560 mg of (R)-2-propanoyloxy-1-tosyloxy-hexane as colourless oil, with $[\alpha]_D^{25}$ = +12.3 (c=1, $CHCl_3$), ee = 75%, $^1$H-NMR (200 MHz, $CDCl_3$) $\delta$ (ppm): 0.85 (3H, t), 1.1 (3H, t), 1.3 (4H, m), 1.6 (2H, m), 2.3 (2H, q), 2.45 (3H, s), 4.0 (2H, d), 4.9 (1H, m), 7.3 and 7.7 (4H, 2d).

The enantiomeric excess of the above compounds was determined as described in example 1.

EXAMPLE 5

Separation of the enantiomers of 3-chloro-1-tosyloxy-propan-2-ol

1 g of propionic anhydride and 2.45 g of Celite® 577, containing 700 mg of Lipasis P enzyme immobilized as described in example 1, are added to 2 g of (R,S)-3-chloro-1-tosyloxy-propan-2-ol dissolved in 50 ml of toluene.

The mixture is kept under vigorous stirring for 3 hours (56% conversion), and the supported enzyme is then separated by filtration.

The organic mixture is dried on sodium sulphate and evaporated at reduced pressure. The residue thus obtained is separated on a silica gel column using hexane-ethyl acetate (first 9:1 V/V and then 7:3 V/V) as eluent.

In this way there are obtained 830 mg of (S)-3-chloro-1-tosyloxy-propan-2-ol as colourless oil with $[\alpha]_D^{25}$ = +4.43 (c=1, $CHCl_3$), ee = ≧95%, $^1$H-NMR (200 MHz, $CDCl_3$), $\delta$ (ppm): 2.44 (3H, s), 2.95 (1H, s), 3.52 - 4.32 (5H, m), 7.3 and 7.75 (4H, 2d); and 1.1 g of (R)-3-chloro-1-tosyloxy-2-propanoyloxy-propane as colourless oil with $[\alpha]_D^{25}$ = +4.8 (c=1, $CHCl_3$), ee = 54%, $^1$H-NMR (200 MHz, $CDCl_3$) $\delta$ (ppm): 1.1 (3H, t), 2.3 (2H, q), 2.44 (3H, s), 3.6 (2H, m), 4.25 (2H, d), 5.1 (1H, m), 7.3 and 7.75 (4H, 2d).

The enantiomeric excess of these compounds was determined as in example 1.

EXAMPLE 6

Separation of the enantiomers of 1-tosyloxybutan-3-ol

2.5 g of Celite® 577 containing 750 mg of Lipasis P enzyme immobilized as in example 1 and 2 g of propionic anhydride are added to 2.5 g of (R,S)-1-tosyloxybutan-3-ol dissolved in 60 ml of toluene.

The mixture is stirred vigorously at 25°C and the reaction is followed by HPLC using a Chiralcel® OD-Daicel® column.

After 24 hours there is a 60% conversion; at this stage the enzyme is filtered and the recovered organic phase is washed with a 5% aqueous solution of sodium carbonate. The organic phase, after having been dried on sodium sulphate, is evaporated at reduced pressure and the residue is subjected to chromatography on a silica gel column, using a mixture of hexane-ethyl acetate (90/10 V/V) as eluent.

Obtained are 1.7 g of (R)-1-tosyloxy-3-propanoyloxy butane as colourless oil with $[\alpha]_D^{25}$ = -14.5 (c=1, chloroform), ee = 55%, $^1$H-NMR (200 MHz, $CDCl_3$), $\delta$ (ppm): 1.1 (3H, t), 1.2 (d, 3H), 1.9 (q, 2H), 2.2 (q, 2H), 2.4 (s, 3H), 4 (m, 2H), 4.9 (m, 1H), 7.3 (d, 2H), 7.7 (d, 2H); and 760 mg of (S)-1-tosyloxybutan-3-ol as colourless oil, with $[\alpha]_D^{25}$ = +15.1 (c=1, chloroform), ee = 97%.

The enantiomeric excess was determined by HPLC (Chiralcel® OD-Daicel® column); eluent for the alcohol: hexane-isopropanol 90/10 V/V; for the ester: hexane-isopropanol 96/4 V/V.

**Claims**

**1.** Process for the enzymatic separation (resolution) of racemic mixtures of tosyloxy-alkanols of general formula (I)

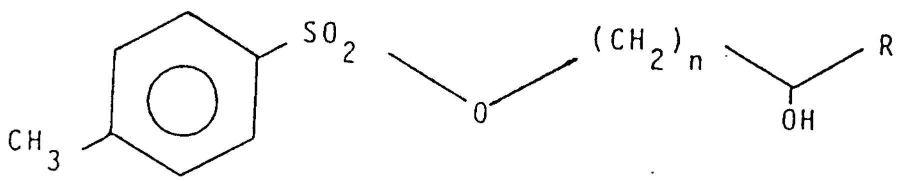

(I)

wherein R represents an alkyl, haloalkyl or $C_2$-$C_{10}$ alkenyl group, and n is 1 or 2; comprising the reaction of said racemic mixtures, in an organic solvent, with an anhydride of a saturated aliphatic acid of general formula (II):

$(R^1\text{-CO})_2O$ (II)

wherein $R^1$ represents a $C_1$-$C_6$ alkyl group; in the presence of a lipase deriving from Pseudomonas or Cromobacterium which is capable of selectively causing the esterification reaction of the (R) isomer, leaving the corresponding (S) isomer basically unchanged.

**2.** Process according to claim 1, wherein said anhydride is selected from acetic anhydride and propionic anhydride.

**3.** Process according to any one of claims 1 and 2, wherein said organic solvent is selected from aromatic hydrocarbons, halogenated aliphatic hydrocarbons, and mixtures thereof.

**4.** Process according to any one of claims 1 to 3, wherein the molar ratio of anhydride of formula (II) to racemic alcohol of formula (I) ranges from 0.6:1 to 5:1.

**5.** Process according to any one of claims 1 to 4, wherein the weight ratio of enzyme to substrate of formula (I) ranges from 1:1 to 1:100.

**6.** Process according to any one of claims 1 to 5, wherein the concentration of racemic alcohol of formula (I) in the reaction mixture ranges from 0.01 M to 2 M.

**7.** Process according to any one of claims 1 to 6, wherein the operating temperature ranges from 0 to 50 °C.

**8.** Process according to any one of claims 1 to 7, wherein said lipase is selected from Lipasis LPL from Pseudomonas aeruginosa, Lipasis P from Pseudomonas fluorescens, Lipasis CES from Pseudomonas sp. and Lipasis from Cromobacterium.

**9.** Process according to any one of claims 1 to 8, wherein said enzyme is immobilized on a support, preferably of the porous kind with a large surface area.

**10.** Process according to claim 9 wherein said support is selected from Celite, porous glass and silica.